# EUROPEAN PATENT SPECIFICATION

(11) **EP 1 413 305 B1**
(45) Date of publication and mention of the grant of the patent: **31.05.2006**
(21) Application number: 02745440.4
(22) Date of filing: 01.07.2002
(51) Int. Cl.: A61K 31/415, A61K 31/4155, A61P 13/10

(54) **ARYL (OR HETEROARYL) AZOLYLCARBYNOL DERIVATIVES FOR THE TREATMENT OF URINARY INCONTINENCE**
ARYL- (ODER HETEROARYL-) AZOLYLCARBYNOLDERIVATVE ZUR BEHANDLUNG VON HARNINKONTINENZ
DERIVES D'ARYL(OU HETEROARYL)AZOLYLCARBINOLES UTILISES DANS LE TRAITEMENT DE L'INCONTINENCE URINAIRE

(30) Priority: 06.07.2001 ES 200101587
(43) Date of publication of application: 28.04.2004
(73) Proprietor: LABORATORIOS DEL DR. ESTEVE, S.A., 08041 Barcelona (ES)
(72) Inventor: MERCE-VIDAL, Ramon, E-08041 Barcelona (ES); ANDALUZ-MATARO, Blas, E-08041 Barcelona (ES); FRIGOLA-CONSTANSA, Jordi, E-08041 Barcelona (ES)
(74) Representative: Carpintero Lopez, Francisco
(86) International application number: PCT/ES2002/000326
(87) International publication number: WO 2003/004022

(56) References cited:
- ES-A- 2 137 136
- ES-A- 2 150 353
- ES-A- 2 150 378
- US-A- 4 416 894

## Description

### Field of the invention

The present invention refers to the use of derivatives of aryl (or heteroaryl) azolylcarbinols of general formula (I), and their physiologically acceptable salts, as medicinal products for human and/or animal therapeutics for the treatment of urinary incontinence.

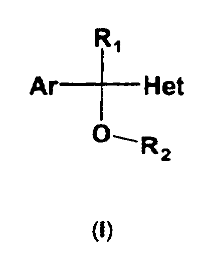

### Background of the invention

Urination is a function of the lower urinary tract that is defined as discharge of urine through the urethra. Urination is considered to be normal in an adult when it is voluntary, continuous, complete, satisfactory, interruptible, spaced out in time (at socially acceptable intervals), without causing abdominal pressure, without urgency, and only occasional at night.

Urinary incontinence, a urinary disorder, is defined as the involuntarily discharge of urine, which can be demonstrated objectively. This functional disorder of bladder is a health problem of increasing social and hygienic relevance for the population that suffers from it. According to our data, urinary incontinence occurs in approximately 1.5 to 5% of men and 10 to 30% of women in the population between 15 and 64 years old. However, if we select the non-hospitalised population sector over 60 years old, the prevalence ranges from 15% to 35% of this population. On the other hand, when hospitalised patients over 60 years old are studied, the incidence is higher. Urinary incontinence affects approximately 2 million of the Spanish population.

Urinary incontinence can be considered as a symptom, sign or pathological condition. The following is one of the possible classifications of this functional disorder.

**Imperative micturition or urge incontinence.** This is when the involuntary discharge of urine is accompanied by an intense desire to urinate (urgency). This can be separated into motor urgency incontinence or sensitive urgency incontinence. Motor urgency incontinence is associated with hyperactivity of the detrusor muscle and/or reduced distensibility of the detrusor. Hyperactivity is characterised by involuntary contractions of the detrusor during the filling stage, either spontaneous or provoked, that the patient cannot totally suppress. Hyperactivity of the detrusor muscle can occur when there is obstruction of the exiting urinary flow, inflammation and conditions in which the bladder is irritated, or it can be of unknown aetiology (idiopathic).

**Hyperreflexia,** is described as a condition that presents uncontrolled contractions of the detrusor muscle associated with neurological disorders such as multiple sclerosis or plaque sclerosis, sequelae of medular traumatisms or Parkinson's disease.

**Urinary stress incontinence,** due to a defective urethral closure mechanism, there is involuntary discharge of urine in the absence of detrusor contraction that occurs when the intravesical pressure exceeds the pressure in the urethra. Involuntary discharge occurs when some physical exertion is made such as jumping, coughing, going down stairs etc. One additional factor can be due to structural changes in the urethra due to menopausal hypooestrogenia.

**Mixed incontinence,** this term refers to the existence of both urgency incontinence and stress incontinence.

The therapeutic options for urinary incontinence depend on the type of incontinence. In urgency incontinence, the first and most effective therapeutic approach is pharmacological treatment accompanied by a series of hygiene regulations and patient education, with secondary approaches including other therapies such as maximum electrical stimulation or surgical treatment. Conservative measures such as pelvic floor exercises and surgical treatment, as a first option, are reserved for stress incontinence.

Pharmacological treatment of urinary urgency incontinence and of hyperreflexia is aimed at reducing activity of the detrusor muscle and increasing the bladder capacity. In cases of stress incontinence, the treatment is aimed at increasing resistance to urinary discharge.

The drugs used to treat urinary incontinence include a wide therapeutic range of drugs from different pharmacological groups with different action mechanisms [Hattori T., Drug treatment of urinary incontinence. *Drugs of Today,* 1998, 34 (2): 125-138], although there is a great deal of confusion and the clinical efficacy of these has not been completely demonstrated.

In a first group of drugs that have an anticholinergic action, propantheline can be considered as a pure anticholinergic agent. There is also a new drug, tolterodine, that has a selective anticholinergic action but that is not selective for the different subtypes of muscarinic receptors although it does appear to have a selectivity of action that is centred around the urinary bladder (detrusor), salivary glands and human intestine. One of the drugs with an anticholinergic action, oxybutin, is a drug with a mixed action, a moderate anticholinergic agent and is a strong direct muscular relaxant. Oxybutin is now the first drug of choice for this disorder, in spite of its tolerability profile with non-severe but annoying adverse effects such as dry mouth, constipation and drowsiness that, in some cases, can cause the patient to abandon the treatment.

Several tricyclic antidepressants have beneficial effects in patients with detrusor hyperactivity. Imipramine, a drug used in clinical practise, has been shown to be an effective treatment for nocturnal enuresis in children and vesical hyperactivity, for example, in the elderly. Owing to the different adverse events reported for this group of drugs, sometimes of strong intensity (e.g. cardiovascular events), the risk-benefits of this treatment for urination disorders must be studied in certain populations, especially in the elderly.

The α-adrenergic antagonists such as prazosin, terazosin or doxazosin can improve detrusor hyperactivity and symptoms related with detrusor dysfunction in patients with benign prostrate hyperplasia, although the evidence for this effect in hyperactive bladder is currently under discussion and there are no data to support its use in urgency incontinence.

Another therapeutically interesting group corresponds to the β-adrenergics, although there is still little information available about their efficacy. It is known that β-adrenergic stimulation can relax the human bladder in normal conditions. The detrusor muscle, both in normal conditions or in the case of an unstable bladder shows a similar degree of response, relaxation, to an β-agonist drug. The β₂₋adrenergic receptor agonists, such as terbutaline or albuterol, have been shown to be able to increase the bladder capacity. In contrast, efficacy of this drug in the treatment of detrusor hyperactivity has been shown in very few controlled clinical studies and in only a small sample of patients.

In our patents EP 289380 and WO 99/52525 we have described derivatives of carbinols of general formula (I) with analgesic activity,

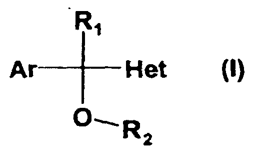

In these compounds of general formula (I). Ar represents a benzene ring or a thiophene ring with or without substitutions, R₁ represents a hydrogen atom or a lower alkyl group from C₁ to C₄; R₂ represents a dialkylaminoalkyl or azaheterocyclylalkyl and Het represents an azole with or without substitutions, and their physiologically acceptable salts.

In our patents WO 97/20817, WO 99/02500, WO 99/07684 and WO 99/52525 we have also described several procedures to prepare enantiomerically pure compounds with general formula (I).

We have also discovered now that general formula (I) compounds, and their physiologically acceptable salts, are especially useful for producing drugs, in human or veterinary therapeutics, to cure or relieve urinary incontinence.

### Detailed description of the invention

The present invention refers to the use of derivatives of aryl (or heteroaryl) azolylcarbinols of general formula (I) 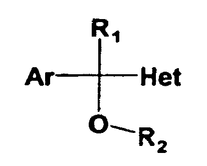 in which
Ar represents a phenyl radical or a thienyl radical, without substitutions or optionally with 1,2 or 3 equal or different substituents selected from a group comprised of fluorine, chlorine, bromine, methyl, trifluoromethyl and methoxy;
R₁ represents a hydrogen atom or a lower alkyl group from C₁ to C₄;
R₂ represents a dialkyl (C₁-C₄) aminoalkyl (C₂-C₃) radical, or azaheterocyclylalkyl (C₂-C₃); and
Het represents an azole, i.e. a five-membered nitrogenated aromatic heterocycle that contains from one to three nitrogen atoms, without substitutions or optionally with substitutions by 1 or 2 equal or different substituents selected from a group comprised of fluorine, chlorine, bromine and methyl;
or one of its physiologically acceptable salts,
In the production of a drug to treat urinary incontinence, in mammals, including man, especially in patients that present an urgency or hyperreflexive incontinence.

The term "lower alkyl group from C₁ to C₄" represents a linear or branched chain radical derived from a saturated hydrocarbon of 1 to 4 carbon atoms, such as methyl, ethyl, propyl, isopropyl, butyl, isobutyl, *sec*-butyl and *tert*-butyl.

The term "dialkyl(C₁-C₄)aminoalkyl (C₂-C₃), or azaheterocyclylalkyl (C₂-C₃)" represents an alkyl radical with two or three carbon atoms joined to a dialkyl (C₁-C₄) amine or to a cyclic amine, such as, for example, dimethylaminoethyl, dimethylaminopropyl, diethylaminoethyl, piperidinylethyl, morpholinylpropyl, pirrolidinylalkyl, etc.

Illustrative examples of compounds included in the present invention include:
(±)-5-{α-[2-(dimethylamine)ethoxy]benzyl}-1-methyl-1*H*-pirazole.
(±)-5-{α-[2-(dimethylamine)ethoxy]benzyl}-1-methyl-1*H*-pirazole citrate.
(+)-5-{α-[2-(dimethylamine)ethoxy]benzyl}-1-methyl-1*H*-pirazole.
(-)-5-{α-[2-(dimethylamine)ethoxy]benzyl}-1-methyl-1*H*-pirazole.
(+)-5-{α-[2-(dimethylamine)ethoxy]benzyl}-1-methyt-1*H*-pirazole citrate.
(-)-5-{α-[2-(dimethylamine)ethoxy]benzyl}-1-methyl-1*H*-pirazole.
(±)-5-{α-[2-(dimethylamine)ethoxy]-2-thienylmethyl}-1-methyl-1*H*-pirazole.
(±)-5-{α-[2-(dimethylamine)ethoxy]-2-thienylmethyl}-1-methyl-1*H*-pirazole citrate.
(+)-5-{α-[2-(dimethylamine)ethoxy]-2-thienylmethyl}-1-methyl-1*H*-pirazole.
(-)-5-{α-[2-(dimethylamine)ethoxy]-2-thienylmethyl}-1-methyl-1*H*-pirazole.
(+)-5-{α-[2-(dimethylamine)ethoxy]-2-thienylmethyl}-1-methyl-1*H*-pirazole citrate.
(-)-5-{α-[2-(dimethylamine)ethoxy]-2-thienylmethyl}-1-methyl-1*H*-pirazole citrate.

The compounds of general formula (I) can be synthesised according to the procedures described in patents EP 289380 or WO 99/52525. The compounds of general formula (I) have a stereogenic centre and the invention refers both to the use of a pure enantiomer and to the use of a mixture of enantiomers. The enantiomers can be prepared by any of the procedures described in our patents WO 97/20817, WO 99/02500, WO 99/07684 or WO 99/52525.

In the present invention, the activity of general formula (I) compounds has been demonstrated in processes of hyperactivity of the urinary bladder, and they are, therefore, useful in urinary incontinence due to hyperreflexive detrusor activity and urgency incontinence.

Next, some of the properties are indicated, which form the object of the invention for (±)-5-{α-[2-(dimethylamine)ethoxy]benzyl}-1-methyl-1*H*-pirazole citrate (Example 1), of formula

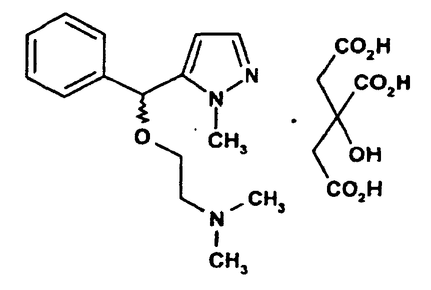

The examples in the following section describing pharmacological trials are merely illustrative and the invention cannot be considered in any way as being restricted to these applications.

The activity of Example 1 has been studied against cyclophosphamide-induced inflammation of the urinary bladder in rats. Cyclophosphamide is an effective form of treatment for several diseases including cancer. One possible side effect of this product is acute inflammation of the bladder. Its activity is based on conversion of the active metabolite in the liver.

Treatment with cyclosphosphamide can give rise to several complications of adverse effects including urinary bladder cystitis, that is mainly due to another cyclophosphamide metabolite, acroleine.

It is known that cyclophosphamide-induced cystitis is due to direct contact of acroleine with the urothelium, although the precise mechanism of this inflammatory response is largely unknown. One of the manifestations of inflammatory response is extravasation of plasma in the urinary bladder. For this reason, the activity of Example 1 against cystitis induced by cyclophosphamide in the urinary bladder of the rat has been studied and its effect on extravasation of plasmatic proteins in the urinary bladder determined.

Extravasation of plasmatic proteins has been measured by the permeability technique using Evan's blue dye, described by A. Saria and J.M. Lundberg (J. Neurosci. Methods 8 : 41-49, 1983). In the first place, the rats were administered Example 1 (80 mg/kg, ip) or vehicle. Five minutes later they were administered cyclophosphamide (150 mg/kg, ip). Three and a half hours later the rats were anaesthetized with urethane (1.2 gr/kg, ip), the jugular vein was cannulated and Evan's blue dye dissolved in H₂O (50 mg/2.5 ml) was administered at a dose of 50 mg/kg, iv. Fifteen minutes after injecting the dye the rats were exsanguinated by infusing 50 ml of saline solution (0.9 %) at 37°C, by cardiac puncture. Then, the urinary bladder was removed, weighed and its contents of Evan's blue dye was determined by spectrophotometry (at 620 mm) after its extraction in a known volume of formamide at 60°C for 24 hours. Extravasation of the plasmatic protein was expressed as the contents of Evan's blue dye in microgrammes per gramme of tissue.

The results obtained show that Example 1 significantly inhibits, by more than 75%, the extravasation of plasmatic protein. Therefore, the protective effect of Example 1 in inflammatory conditions of the urinary bladder is evident, taking as an example all processes similar to cyclophosphamide induced cystitis.

| Group | N° of rats | Extravasation of plasmatic protein µg Evans blue/ g. tissue | |
|---|---|---|---|
| Control | 10 | 25 | |
| Cyclophosphamide | 10 | 437 | |
| (150 mg/kg, ip) | | | |
| Cyclophosphamide + Example 1 | 10 | 125 | (Inhibition = 75.7 %) |
| (150 mg/kg + 80 mg/kg, ip) | | | |

Taking into account its good pharmacodynamic properties, derivatives of aryl(o heteroaryl)azolylcarbinol, according to the invention, can be used satisfactorily in human and animal therapeutics to cure and relieve urinary incontinence.

In human therapeutics, the dose administered of the compounds of the invention depends on the severity of the infection to be treated. It is normally between 50 and 400 mg/day. The compounds of the invention are administered for example in the form of capsules or tablets.

In the following section, as an example, specific pharmaceutical formulae of the compounds of the invention are specified.

### Example of formula for injectable (im/iv):

| | |
|---|---|
| Citrate of (±)-5-{α-[2-(dimethytamine)ethoxy]benzyl}-1-methyl-1*H*-pirazole | 50 mg |
| 0.1 N Sodium hydroxide | c.s. pH 6 |
| Water for injection c.s.p. | 1 ml |

### Example of formula for tablet

| | |
|---|---|
| (±)-5-{α-[2-(dimethylamine)ethoxy]benzyl}-1-methyl-1*H*-pirazole citrate | 400 mg |
| Sodium croscarmelose (Ac-Di-Sol) | 32 mg |
| Coltoidal silica dioxide (Aerosyl 200) | 8 mg |
| Magnesium stearate, NF | 16 mg |
| Povidone K-30 | 40 mg |
| Microcrystalline cellulose (Avicel PH-102) | 146 mg |
| Lactose monohydrate (Farmatose 200M) | 158 mg |
| Total | 800 mg |

### Example of formula per capsule

| | |
|---|---|
| (±)-5-{α-[2-(dimethylamine)ethoxy]benzyl}-1-methyl-1*H*-pirazole citrate | 200.0 mg |
| Colloidal silica dioxide | 0.8 mg |
| Magnesium stearate | 2.4 mg |
| Lactose | 276.8 mg |
| Total | 480 mg |

## Claims

1. Use of a derivative of aryl (or heteroaryl)azolylcarbinol of general formula (I) 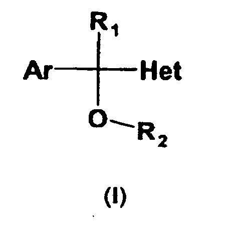 in which
Ar represents a phenyl radical or a thienyl radical, with no substitutions or optionally with 1, 2 or 3 equal or different substituents selected from the group comprised of fluorine, chlorine, bromine, methyl, trifluoromethyl and methoxy;
R₁ represents a hydrogen atom or a lower alkyl group from C₁ to C₄;
R₂ represents a dialkyl(C₁-C₄)aminoalkyl (C₂-C₃), or azaheterocyclylalkyl (C₂₋C₃) radical; and
Het represents a five-membered nitrogenated aromatic heterocycle that contains one to three nitrogen atoms, without substitutions or optionally substituted by 1 or 2 equal or different substituents selected from a group comprised by fluorine, chlorine, bromine and methyl;
or one of its physiologically acceptable salts,
in the production of a drug to treat urinary incontinence in mammals, including man.

2. Use according to Claim 1, of a compound of general formula (I), in which R₁ is selected from a hydrogen atom or from the group comprised by methyl, ethyl, propyl, isopropyl, butyl, isobutyl, *sec*-butyl and *tert*-butyl, in the production of a drug for the treatment of urinary incontinence in mammals, including man.

3. Use, according to Claim 1, of a compound of general formula (I), in which R₂ is selected from among a group comprised of dimethylaminoethyl, dimethylaminopropyl, diethylaminoethyl, piperidinylethyl, morpholinylpropyl and pirrolidinylethyl, in the production of a drug to treat urinary incontinence in mammals, including man.

4. Use according to Claim 1, of a compound of general formula (I) selected from among a group comprised by:
(±)-5-{α-[2-(dimethylamine)ethoxy]benzyl}-1-methyl-1*H*-pirazole;
(±)-5-{α-[2-(dimethylamine)ethoxy]benzyl}-1-methyl-1*H*-pirazole citrate;
(+)-5-{α-[2-(dimethylamine)ethoxy]benzyl}-1-methyl-1*H*-pirazole;
(-)-5-{α-[2-(dimethylamine)ethoxy]benzyl}-1-methyl-1*H*-pirazole;
(+)-5-{α-[2-(dimethylamine)ethoxy]benzyl}-1-methyl-1*H*-pirazole citrate;
(-)-5-{α-[2-(dimethylamine)ethoxy]benzyl}-1-methyl-1*H*-pirazole citrate;
(±)-5-{α-[2-(dimethylamine)ethoxy]-2-thienylmethyl}-1-methyl-1*H*-pirazole;
(±)-5-{α-[2-(dimethylamine)ethoxy]-2-thienylmethyl}-1-methyl-1*H*-pirazole citrate;
(+)-5-{α-[2-(dimethylamine)ethoxy]-2-thienylmethyl}-1-methyl-1*H*-pirazole;
(-)-5-{α-[2-(dimethylamine)ethoxy]-2-thienylmethyl}-1-methyl-1*H*-pirazole;
(+)-5-{α-[2-(dimethylamine)ethoxy]-2-thienylmethyl}-1-methyl-1*H*-pirazole citrate; and
(-)-5-{α-[2-(dimethylamine)ethoxy]-2-thienylmethyl}-1-methyl-1*H*-pirazole citrate;
in the production of a drug to treat urinary incontinence in mammals, including man.

## Patentansprüche

1. Verwendung eines Derivats von Aryl (oder Heteroaryl)azolylcarbinol der allgemeinen Formel (I) 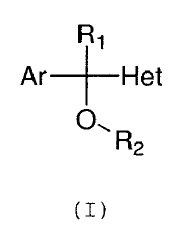 worin
Ar einen Phenylrest oder einen Thienylrest bedeutet, ohne Substitutionen oder optional mit 1, 2 oder 3 gleichen oder unterschiedlichen Substituenten, gewählt aus der Gruppe, umfassend Fluor, Chlor, Brom, Methyl, Trifluormethyl und Methoxy;
R₁ repräsentiert ein Wasserstoffatom oder eine Niederalkylgruppe von C₁ bis C₄;
R₂ repräsentiert einen Dialkyl(C₁-C₄)aminoalkyl(C₂-C₃)- oder Azaheterocyclylalkyl(C₂-C₃)-Rest; und
Het repräsentiert einen fünfgliedrigen mit Stickstoff versehenen aromatischen Heterozyklus, der ein bis drei Stickstoffatome enthält, ohne Substitutionen oder optional durch 1 oder 2 gleiche oder unterschiedliche Substituenten substituiert, gewählt aus einer Gruppe, umfassend Fluor, Chlor, Brom und Methyl;
oder eines seiner physiologisch akzeptablen Salze, für die Herstellung eines Arzneimittels zur Behandlung einer Harninkontinenz bei Säugern, einschließlich dem Menschen.

2. Verwendung gemäß Anspruch 1 einer Verbindung der allgemeinen Formel (I), worin R₁ gewählt ist aus einem Wasserstoffatom oder aus der Gruppe, umfassend Methyl, Ethyl, Propyl, Isopropyl, Butyl, Isobutyl, sec-Butyl und tert-Butyl, für die Herstellung eines Arzneimittels für die Behandlung einer Harninkontinenz bei Säugern, einschließlich dem Menschen.

3. Verwendung gemäß Anspruch 1 einer Verbindung der allgemeinen Formel (I), worin R₂ gewählt ist aus einer Gruppe, umfassend Dimethylaminoethyl, Dimethylaminopropyl, Diethylaminoethyl, Piperidinylethyl, Morpholinylpropyl und Pyrrolidinylethyl für die Herstellung eines Arzneimittels zur Behandlung einer Harninkontinenz bei Säugern, einschließlich dem Menschen.

4. Verwendung gemäß Anspruch 1 einer Verbindung der allgemeinen Formel (I), gewählt aus einer Gruppe, umfassend:
(±)-5-{-[2-(Dimethylamin)ethoxy]benzyl}-1-methyl-1H-pyrazol;
(±)-5-{-[2-(Dimethylamin)ethoxy]benzyl}-1-methyl-1H-pyrazolzitrat;
(+)-5-{-[2-(Dimethylamin)ethoxy]benzyl}-1-methyl-1H-pyrazol;
(-)-5-{-[2-(Dimethylamin)ethoxy]benzyl}-1-methyl-1H-pyrazol;
(+)-5-{-[2-(Dimethylamin)ethoxy]benzyl}-1-methyl-1H-pyrazolzitrat;
(-)-5-{-[2-(Dimethylamin)ethoxy]benzyl}-1-methyl-1H-pyrazolzitrat;
(±)-5-{-[2-(Dimethylamin)ethoxy]-2-thienylmethyl}-2-methyl-1H-pyrazol;
(±)-5-{-[2-(Dimethylamin)ethoxy]-2-thienylmethyl}-1-methyl-1H-pyrazolzitrat;
(+)-5-{-[2-(Dimethylamin)ethoxy]-2-thienylmethyl}-1-methyl-1H-pyrazol;
(--)-5-{-[2-(Dimethylamin)ethoxy]-2-thienylmethyl}-1-methyl-1H-pyrazol;
(+)-5-{-[2-(Dimethylamin)ethoxy]-2-thienylmethyl}-1-methyl-1H-pyrazolzitrat; und
(--)-5-{-[2-(Dimethylamin)ethoxy]-2-thienylmethyl}-1-methyl-1H-pyrazolzitrat;
bei der Herstellung eines Heilmittels zur Behandlung einer Harninkontinenz bei Säugern, einschließlich dem Menschen.

## Revendications

1. Utilisation d'un dérivé d'un aryl-azolylcarbinol ou hétéroaryl-azolylcarbinol- de formule générale (I): 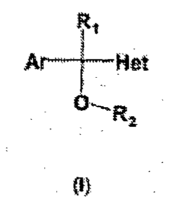 dans laquelle:
Ar représente un radical phényl ou un radical thiènyl, non substitué ou éventuellement substitué par 1, 2 ou 3 substituants, identiques ou différents, choisis dans le groupe comprenant les atomes de fluor, de chlore ou de brome et les radicaux méthyl, trifluorométhyl ou méthoxy;
R₁ représente un atome d'hydrogène ou un radical alkyl inférieur en C₁ à C₄;
R₂ représente un radical (C₁-C₄)-dialkyl-(C₂-C₃)-aminoalkyl, ou un radical azahétérocyclyl-(C₂ à C₃)-alkyl;
et
Het représente un hétérocycle aromatique azoté à 5 atomes, dont 1 à 3 de ces atomes sont des atomes d'azote, non substitué ou éventuellement substitué par 1 ou 2 substituants, identiques ou différents, choisis dans le groupe comprenant les atomes de fluor, de chlore ou de brome et le radical méthyl;
ou d'un de ses sels pharmaceutiquement acceptables,
pour la production d'un médicament pour traiter l'incontinence urinaire chez les mammifères, y compris l'homme.

2. Utilisation selon la revendication 1, d'un composé de formule générale (I), dans laquelle R₁ est choisi dans le groupe comprenant l'atome d'hydrogène et les radicaux méthyl, éthyl, propyl, isopropyl, butyl, isobutyl, sec.-butyl, et tert.-butyl, pour la production d'un médicament pour traiter l'incontinence urinaire chez les mammifères, y compris l'homme.

3. Utilisation selon la revendication 1, d' un composé de formule générale (I), dans laquelle R₂ est choisi dans le groupe comprenant les radicaux diméthylaminoéthyl, diméthylaminopropyl, diéthylaminoéthyl, pipéridinyléthyl, morpholinylpropyl et pirrolidinyléthyl, pour la production d'un médicament pour traiter l'incontinence urinaire chez les mammifères, y compris l'homme.

4. Utilisation selon la revendication 1, d'un composé de formule générale (I), choisi dans le groupe comprenant:
le (±)-5-{α-[2-(diméthylamine)-éthoxy]-benzyl}-1-méthyl-1H-pirazole;
le citrate de (±)-5-{α-[2-(diméthylamine)-éthoxy]-benzyl}-1-méthyl-1H-pirazole;
le (+)-5-{α-[2-(diméthylamine)-éthoxy]-benzyl}-1-méthyl-1H-pirazole;
le (-)-5-{α-[2-(diméthylamine)-éthoxy]-benzyl}-1-méthyl-1H-pirazole;
le citrate de (+)-5-{α-[2-(diméthylamine)-éthoxy]-benzyl}-1-méthyl-1H-pirazole;
le citrate de (-)-5-{α-[2-(diméthylamine)-éthoxy]-benzyl}-1-méthyl-1H-pirazole;
le (±)-5-{α-[2-(diméthylamine)-éthoxy]-2-thiénylméthyl}-1-méthyl-1H-pirazole;
le citrate de (±)-5-{α-[2-(diméthylamine)-éthoxy]-2-thiénylméthyl}-1-méthyl-1H-pirazole;
le (+)-5-{α-[2-(diméthylamine)-éthoxy]-2-thiénylméthyl}-1-méthyl-1H-pirazole;
le (-)-5- {α-[2-(diméthylamine)-éthoxy]-2-thiénylméthyl}-1-méthyl-1H-pirazole;
le citrate de (+)-5-{α-[2-(diméthylamine)-éthoxy]-2-thiénylméthyl}-1-méthyl-1H-pirazole;
et
le citrate de (-)-5-{α-[2-(diméthylamine)-éthoxy]-2-thiénylméthyl}-1-méthyl-1H-pirazole
pour la production d'un médicament pour traiter l'incontinence urinaire chez les mammifères, y compris l'homme.
